# EUROPEAN PATENT SPECIFICATION

(11) **EP 4 048 658 B1**
(45) Date of publication and mention of the grant of the patent: **03.06.2026**
(21) Application number: 20880036.7
(22) Date of filing: 23.10.2020
(51) Int. Cl.: C07D 257/04, A61K 31/41, A61P 3/04, A61P 25/00, A61P 25/04, A61P 25/08, A61P 25/20, A61P 25/22, A61P 25/24, A61P 25/28, A61P 25/30, A61P 25/32, A61P 25/34, A61P 25/36

(54) **PROCESS FOR THE PREPARATION OF 1-PHENYL-2-(2H-TETRAZOL-2-YL)-ETHANONE DERIVATIVES FROM 2-HALO-1-PHENYL-ETHANONE DERIVATIVES AND TETRAZOLE SALT**
VERFAHREN ZUR HERSTELLUNG VON 1-PHENYL-2-(2H-TETRAZOL-2-YL)-ETHANON-DERIVATEN AUS 2-HALO-1-PHENYL-ETHANON-DERIVATIVEN UND TETRAZOL SALZ
PROCÉDÉ DE PRÉPARATION DE DÉRIVÉS DE 1-PHÉNYL-2-(2H-TÉTRAZOL-2-YL)-ÉTHANONE A PARTIR DE DÉRIVÉS DE 2-HALO-1-PHENYL-ETHANONE ET SÉL DE TETRAZOLE

(30) Priority: 24.10.2019 US 201916662547; 24.10.2019 KR 20190133120
(43) Date of publication of application: 31.08.2022
(73) Proprietor: SK Biopharmaceuticals Co., Ltd., Seongnam-si, Gyeonggi-do 13494 (KR)
(72) Inventor: LEE, Kyu Woong, Seongnam-si Gyeonggi-do 13494 (KR); CHA, Kyung Mi, Seongnam-si Gyeonggi-do 13494 (KR); YEOM, Su Yeon, Seongnam-si Gyeonggi-do 13494 (KR); WOO, Ji Seon, Seongnam-si Gyeonggi-do 13494 (KR)
(74) Representative: Vossius & Partner Patentanwälte Rechtsanwälte mbB
(86) International application number: PCT/KR2020/014599
(87) International publication number: WO 2021/080380

(56) References cited:
- WO-A1-2010/150946
- WO-A2-2011/046380
- JP-A- S58 140 083
- TRIFONOV R E ET AL: "Protolytic equilibria in tetrazoles", RUSSIAN JOURNAL OF ORGANIC CHEMISTRY, NAUKA/INTERPERIODICA, MO, vol. 42, no. 11, 2006, pages 1585 - 1605, XP019454156, ISSN: 1608-3393, DOI: 10.1134/S1070428006110017
- KRYLOV A. S., DOGADINA A. V., TRIFONOV R. E.: "5-phenyl-2H-tetrazol-2-ylmethyl ketones in the synthesis of tetrazolylalkanols and tetrazolyl(hydroxy)alkylphosphonates", RUSSIAN JOURNAL OF ORGANIC CHEMISTRY., M A I K NAUKA - INTERPERIODICA, RU, vol. 50, no. 6, 1 June 2014 (2014-06-01), RU, pages 892 - 894, XP055805727, ISSN: 1070-4280, DOI: 10.1134/S1070428014060219
- ROSTOM, S.A.F. ; ASHOUR, H.M.A. ; RAZIK, H.A.A.E. ; FATTAH, A.E.F.H.A.E. ; EL-DIN, N.N.: "Azole antimicrobial pharmacophore-based tetrazoles: Synthesis and biological evaluation as potential antimicrobial and anticonvulsant agents", BIOORGANIC & MEDICINAL CHEMISTRY, ELSEVIER, AMSTERDAM, NL, vol. 17, no. 6, 15 March 2009 (2009-03-15), AMSTERDAM, NL, pages 2410 - 2422, XP025981938, ISSN: 0968-0896, DOI: 10.1016/j.bmc.2009.02.004
- DANIEL SPINKS, LEAH S. TORRIE, STEPHEN THOMPSON, JUSTIN R. HARRISON, JULIE A. FREARSON, KEVIN D. READ, ALAN H. FAIRLAMB, PAUL G. W: "Design, Synthesis and Biological Evaluation of Trypanosoma brucei Trypanothione Synthetase Inhibitors", CHEMMEDCHEM, WILEY-VCH, vol. 7, no. 1, 2 January 2012 (2012-01-02), pages 95 - 106, XP055168733, ISSN: 18607179, DOI: 10.1002/cmdc.201100420

## Description

### Technical Field

The present disclosure relates to a method for preparing aryl 2-tetrazol-2-yl ketone of Formula 1a with improved selectivity. 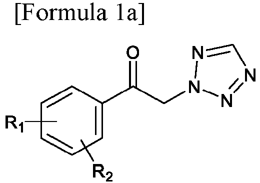 wherein R₁ and R₂ are as defined herein.

### Background Art

Carbamic acid (R)-1-aryl-2-tetrazolyl-ethyl ester (hereinafter also referred to as "carbamate compound") is useful in the treatment of CNS disorders, particularly anxiety, depression, convulsions, epilepsy, migraine, manic depression, drug abuse, smoking, attention deficit hyperactivity disorder (ADHD), obesity, sleep disorders, neuropathic pain, stroke, cognitive impairment, neurodegeneration, muscle spasm due to stroke and the like, according to its anticonvulsant effects.

The carbamate compound is prepared from a compound of Formula 1a, which is obtained from a substitution reaction of a compound of Formula 2 and a compound of Formula 3, as an intermediate. Conventionally, a base is added to the compound of Formula 2, and a tetrazole solution is then added thereto to carry out a substitution reaction. However, in this case, the compound of Formula 1b, which is a positional isomer thereof, in addition to the desired compound of Formula 1a, is obtained together as a mixture by the substitution reaction (WO 2011/046380).

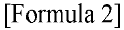

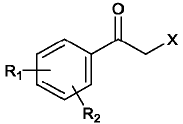

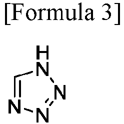

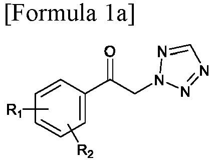

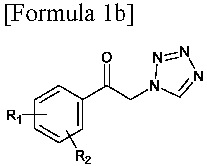

(In the Formulas, R₁ and R₂ are each independently selected from the group consisting of hydrogen, halogen, perfluoroalkyl having 1 to 8 carbon atoms, alkyl having 1 to 8 carbon atoms, thioalkoxy having 1 to 8 carbon atoms and alkoxy having 1 to 8 carbon atoms; and X is a leaving group)

WO 2010/150946 A1 discloses a method for the preparation of carbamic acid (R)-1-aryl-2-tetrazolyl-ethyl ester, comprising the asymmetric reduction of arylketone and the carbamation of alcohol.

Furthermore, when the compound of Formula 2 and the compound of Formula 3 are subjected to the substitution reaction, the reaction selectivity of number 1 nitrogen of the compound of Formula 3 is better than the reaction selectivity of number 2 nitrogen, and thus the compound of Formula 1b is produced with superior selectivity with respect to the compound of Formula 1a (Journal of the Chemical Society, Perkin Transactions 1: Organic and Bio-Organic Chemistry (1972-1999), (7), 1157-63; 1986). Therefore, according to the conventional method, the compound of Formula 1a used for preparing the carbamate compound is less produced than the compound of Formula 1b, and thus the yield is low when preparing the carbamate compound by using the compound of Formula 2 and the compound of Formula 3 as starting materials.

In this regard, there is an alternative method for selectively preparing only a compound of the same form as substituted with number 2 nitrogen by synthesizing a tetrazole ring shape (Chem. Pharm. Bull. 30(9) 3450-3452; 1982). However, there may be problems in that it is difficult to be commercially used since a diazomethane-based material-which has a risk of explosion during the reaction-is used, and 2 equivalents or more of lithium diisopropylamide is used as a raw material.

As such, there is a need for developing a method that the aryl 2-tetrazol-2-yl ketone of Formula 1a may be prepared from the compound of Formula 2 and the compound of Formula 3 with better selectivity than the aryl 2-tetrazol-1-yl ketone of Formula 1b, and as a result, can be commercialized while obtaining the aryl 2-tetrazol-2-yl ketone of Formula 1a and the carbamate compound in high yield.

### Disclosure of Invention

### Technical Problem

The purpose of the present disclosure is to provide a commercially available method capable of improving the productivity of a carbamate compound by more selectively synthesizing aryl 2-tetrazol-2-yl ketone, which is useful as an intermediate of carbamate compound, in large scale.

### Solution to Problem

One aspect of the present invention provides a method for preparing a compound of Formula 1a, which comprises a step of reacting a compound of Formula 3 with a base in a reaction solvent to obtain a salt of a compound of Formula 3; and a step of reacting a compound of Formula 2 with the salt of the compound of Formula 3: 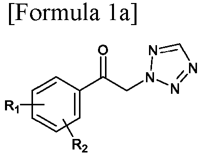 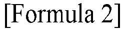 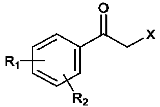 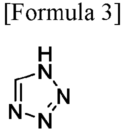 wherein R₁ and R₂ are each independently selected from the group consisting of hydrogen, halogen, perfluoroalkyl having 1 to 8 carbon atoms, alkyl having 1 to 8 carbon atoms, thioalkoxy having 1 to 8 carbon atoms, and alkoxy having 1 to 8 carbon atoms; and X is a leaving group selected from halides and sulfonates.

Another aspect of the present disclosure provides a method for increasing the selectivity of a compound of Formula 1a by using a salt of a compound of Formula 3 in the synthesis of a compound of Formula 1a and a compound of Formula 1b from a compound of Formula 2 and a compound of Formula 3: 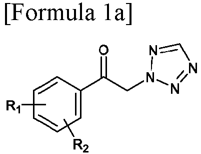 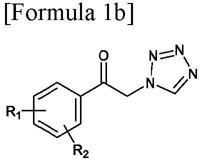 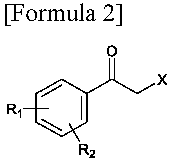 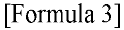 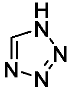 wherein R₁, R₂ and X are the same as defined above.

Another aspect of the present disclosure provides a method of preparing a compound of Formula 4, comprising: (1) reacting a compound of Formula 2 with a salt of a compound of Formula 3; (2) separating the compound of Formula 1a from a mixture obtained by the reaction of step (1); and (3) reducing the compound of Formula 1a separated in step (2) and carbamating the reduced compound of Formula 1a: 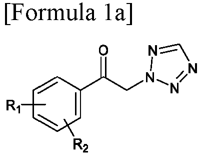 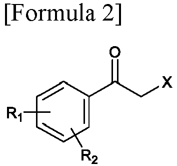 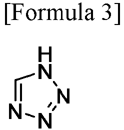 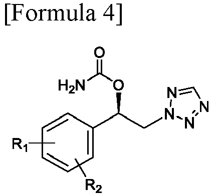 wherein R₁, R₂ and X are the same as defined above.

Another aspect of the present disclosure provides a method for separating a compound of Formula 1a from a mixture comprising a compound of Formula 1a and a compound of 1b by heat treating the mixture comprising the compound of chemical formula 1a and the compound of Formula 1b to produce a compound of Formula 5 and removing this compound: 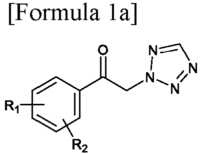 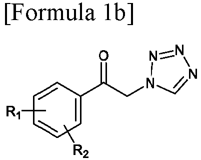 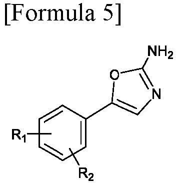 wherein R₁ and R₂ are the same as defined above.

Another aspect of the present disclosure provides a method for preparing a compound of Formula 1a, comprising: reacting a compound of Formula 2 with a salt of a compound of Formula 3; and purifying the reaction product of the salt of the compound of Formula 3 and the compound of Formula 2, wherein the purifying step comprises a crystallizing process or a heat treatment process: 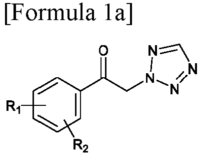 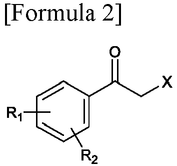 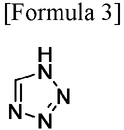 wherein R₁, R₂ and X are the same as defined above.

### Advantageous Effects of Invention

According to the present disclosure, the productivity of carbamate compounds can be improved remarkably as a result by more selectively synthesizing aryl 2-tetrazol-2-yl ketone, in large scale, which is useful as an intermediate of carbamate compound, through a simple process.

### Brief Description of Drawings

FIG. 1 shows HPLC results of the ratio for the mixture of the compound of Formula 1a and the compound of Formula 1b in the reaction mixture produced in Example 1.
FIG. 2 is an ORTEP (Oak Ridge Thermal Ellipsoid Plot) image of the structure of 5-(2-chlorophenyl)oxazol-2-amine obtained in Example 11.
FIG. 3 shows HPLC results of the ratio for the compound of Formula 4 transformed from the compound of Formula 1b and the compound of Formula 1a in the reaction mixture produced in Example 11.

### Modes for the Invention

Hereinafter, the present disclosure is described in detail.

A method for preparing a compound of Formula 1a according to one aspect of the present invention comprises a step of reacting a compound of Formula 3 with a base in a reaction solvent to obtain a salt of a compound of Formula 3; and a step of reacting a compound of Formula 2 with the salt of the compound of Formula 3: 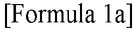 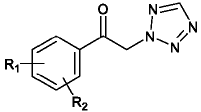 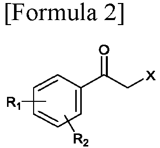 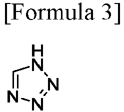
wherein R₁ and R₂ are each independently selected from the group consisting of hydrogen, halogen, perfluoroalkyl having 1 to 8 carbon atoms, alkyl having 1 to 8 carbon atoms, thioalkoxy having 1 to 8 carbon atoms, and alkoxy having 1 to 8 carbon atoms, and more specifically selected from the group consisting of hydrogen, halogen, perfluoroalkyl having 1 to 4 carbon atoms, alkyl having 1 to 4 carbon atoms, and alkoxy having 1 to 4 carbon atoms; and
X is a leaving group selected from halides such as chloride, bromide and the like, and sulfonates such as mesylate, tosylate, 4-nitrophenyl sulfonate and the like.

The salt of the compound of Formula 3 is obtained by reacting the compound of Formula 3 with a base, and the salt may be an inorganic salt or an organic salt.

In one embodiment, the inorganic salt of the compound of Formula 3 may be a metal salt, more specifically an alkali metal salt, and even more specifically a lithium salt, a sodium salt, a potassium salt or a cesium salt.

In one embodiment, the inorganic salt of the compound of Formula 3 may be obtained by reacting the compound of Formula 3 with an inorganic base. The inorganic base may be a metal hydroxide (e.g., lithium hydroxide, sodium hydroxide, potassium hydroxide, etc.) or a metal carbonate (e.g., lithium carbonate, sodium carbonate, potassium carbonate, cesium carbonate, etc.), but is not limited thereto.

In one embodiment, the organic salt of the compound of Formula 3 may be obtained by reacting the compound of Formula 3 with an organic base. The organic base may be an amine compound (e.g., triethylamine, diisopropylethylamine, etc.), but is not limited thereto.

The reaction between the compound of Formula 3 and the base may be carried out at room temperature, and the reaction solvent may be water, acetonitrile, tetrahydrofuran, 2-methyltetrahydrofuran, ethyl acetate, isopropyl acetate, n-butyl acetate, dichloromethane, chloroform, 1,4-dioxane, C₁-C₄ lower alcohol (e.g., methanol, ethanol, propanol, butanol), alone or in combination thereof.

According to one embodiment, the compound of Formula 3 may be reacted with a base in a reaction solvent, and then the salt of the compound of Formula 3 may be separated from the reaction product of the compound of Formula 3 and the base. According to another embodiment, the separated salt of the compound of Formula 3 may be reacted with the compound of Formula 2.

After reacting the compound of Formula 3 with the base, the compound of Formula 2 is added to the reaction product to react with the salt of the compound of Formula 3.

The reaction between the salt of the compound of Formula 3 and the compound of Formula 2 may be carried out at room temperature, and the reaction solvent may be water, acetonitrile, tetrahydrofuran, 2-methyltetrahydrofuran, ethyl acetate, isopropyl acetate, n-butyl acetate, dichloromethane, chloroform, 1,4-dioxane, C₁-C₄ lower alcohol (e.g., methanol, ethanol, propanol, butanol), alone or in combination thereof.

The reaction product of the salt of the compound of Formula 3 and the compound of Formula 2 obtained as described above is a mixture including the compound of Formula 1a and the compound of Formula 1b: 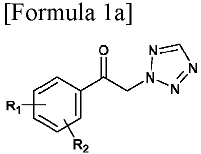 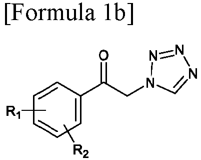 wherein R₁ and R₂ are the same as defined above.

Therefore, in order to separate the compound of Formula 1a and the compound of Formula 1b from each other in the reaction product, the method for preparing the compound of Formula 1a may further include purifying the reaction product of the salt of the compound of Formula 3 and the compound of Formula 2.

In one embodiment, the purifying step may include a crystallization process, and more particularly, the crystallization process may include a first crystallization process and a second crystallization process.

In one embodiment, the crystallization process may be a process in which a first crystallization solvent (for example, water, C₁-C₄ lower alcohol, diethyl ether, tert-butyl methyl ether, isopropyl ether, pentane, hexane, cyclohexane, heptane and a mixture thereof) is added to a reaction product of a salt of a compound of Formula 3 and a compound of Formula 2, the compound of Formula 1b is crystallized, and then filtered and separated ("first crystallization process"), a second crystallization solvent (for example, acetone, acetonitrile, tetrahydrofuran, 2-methyl tetrahydrofuran, ethyl acetate, isopropyl acetate, n-butyl acetate, dichloromethane, chloroform, 1,4-dioxane, C₁-C₄ lower alcohol or a mixture thereof) is added to the remaining filtrate, and the compound of Formula 1a is crystallized, filtered and separated ("second crystallization process").

In one embodiment, a process of washing and concentrating may be further carried out before the first crystallization solvent is added, if necessary.

In one embodiment, the purifying step may include a heat treatment process. Through a heat treatment process, the compound of Formula 1b may be transformed to the compound of Formula 5.

In one embodiment, the heat treatment process may be carried out at a pressure of about 1 atmosphere to 50 atmosphere. The pressure is measured for internal pressure of the reactant and the pressure may depend on the change of the temperature in the reactant.

In one embodiment, the heat treatment process may be carried out at a reaction temperature of 100°C to 250°C, preferably 150°C to 220°C.

In one embodiment, the heat treatment process may be carried out for 10 minutes to 40 hours, preferably 20 minutes to 24 hours. However, the reaction time may be appropriately adjusted according to the reaction temperature.

In one embodiment, the heat treatment process may be a step of heating the reaction product of the salt of the compound of Formula 3 and the compound of Formula 2 to selectively convert only the compound of Formula 1b into the compound of Formula 5 (wherein because the compound of Formula 1a is thermally stable compared to the compound of Formula 1b, the ratio of decomposition or reaction of the compound of Formula 1a due to the heat treatment is extremely low compared to the compound of Formula 1b). The heating may be carried out in the presence of a solvent (e.g., acetone, acetonitrile, tetrahydrofuran, 2-methyl tetrahydrofuran, ethyl acetate, isopropyl acetate, n-butyl acetate, dichloromethane, chloroform, 1,4-dioxane, C₁-C₄ lower alcohol or a mixture thereof).

After the heat treatment process, a process of washing with an acidic aqueous solution may be further carried out. Through a process of washing, the compound of Formula 5, which is transformed from the compound of Formula 1b, is removed.

In one embodiment, the acidic aqueous solution may be a solution of strong acid such as hydrochloric acid or sulfuric acid, or a solution of weak acid such as acetic acid or citric acid, but is not limited thereto. 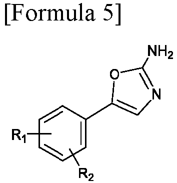 wherein R₁ and R₂ are the same as defined above.

According to the present disclosure, when the compound of Formula 3 is prepared as a salt and then reacted with the compound of Formula 2, the compound of Formula 1a can be obtained with increased selectivity as compared to a conventional method in which the compound of Formula 2, the compound of Formula 3 and the base are simultaneously reacted. Specifically, the ratio of the compound of Formula 1a to the compound of Formula 1b was 4: 6 in the conventional method, but the ratio may become about 6:4 according to the method of the present disclosure.

As the selectivity of the compound of Formula 1a is increased according to the method of the present disclosure compared to the conventional method as described above, the productivities of the compound of Formula 1a and carbamate compound prepared therefrom may also increase remarkably. Specifically, their productivities increase at least about 70%, and more specifically, about 70% to about 100%.

Therefore, another aspect of the present disclosure relates to a method for increasing the selectivity of a compound of Formula 1a by using a salt of a compound of Formula 3 in the synthesis of compounds of Formula 1a and a compound of Formula 1b from a compound of Formula 2 and a compound of Formula 3.

In addition, still another aspect of the present disclosure relates to a preparing a compound of Formula 1a, comprising: reacting a compound of Formula 2 with a salt of a compound of Formula 3; and purifying the reaction product of the salt of the compound of Formula 3 and the compound of Formula 2, wherein the purifying step comprises a crystallizing process or a heat treatment process: 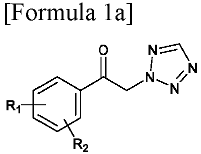 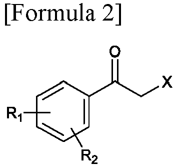 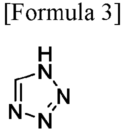 wherein R₁, R₂ and X are the same as defined above.

In addition, still another aspect of the present disclosure relates to a method for preparing a compound of Formula 4, comprising: (1) reacting a compound of Formula 2 with a salt of a compound of Formula 3; (2) separating the compound of Formula 1a from a mixture obtained from the reaction of step (1); and (3) reducing the compound of Formula 1a separated in step 2 and carbamating the reduced compound of Formula 1a: 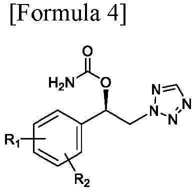 wherein R₁ and R₂ are the same as defined above.

The reaction of a compound of Formula 2 and a compound of Formula 3 is the same as described above.

The separation of a compound of Formula 1a may include the purification step described above.

In the step of reducing and carbamating, the reduction process may be carried out by using an oxidoreductase enzyme that is suspended in a reaction mixture or immobilized in a conventional manner. The enzyme may be used in a completely purified state, a partially purified state or a microbial cell state in which it is expressed. The cells themselves may be in a native state, permeabilized state, or lysed state. It will be understood by those of ordinary skill in the art that when the method of the present disclosure is carried out by the use of enzyme in a cell state allows to highly reduce costs so that it is preferable. Most preferably, the enzyme is expressed in *E. coli* and used as a native cell suspension.

The enzymatic reduction of the compound of Formula 1a may be carried out in a reaction mixture comprising the compound of Formula 1a, an oxidoreductase, NADH or NADPH as a cofactor, a cosubstrate and a suitable buffer. The oxidoreductase can be used to reduce the compound of Formula 1a with high conversion and enantiomeric selectivity using polypeptides having oxidoreductase activity. The enantiomeric excess of the R-configuration alcohol produced in the enantiomeric selective enzymatic reduction is at least about 89%, preferably at least about 95% and most preferably at least about 99%.

In the step of reducing and carbamating, a method for introducing a carbamoyl group may, for example, introduce a carbamoyl group by the use of an inorganic cyanate-organic acid, isocyanate-water or a carbonyl compound-ammonia.

In the carbamation with the inorganic cyanate-organic acid, the (R)-configuration alcohol compound converted from the compound of Formula 1a by the reduction method may be dissolved in an organic solvent-for example, diethylether, tetrahydrofuran, 1,4-dioxane, acetonitrile, dichloromethane, chloroform or a mixture thereof, and then an inorganic cyanate such as sodium cyanate and an organic acid such as methanesulfonic acid or acetic acid, which are 1 to 4 equivalents, may be added thereto, and the reaction may be carried out at a reaction temperature of about -10°C to about 70°C.

In the method of using isocyanate-water, 1 to 4 equivalents of isocyanate-for example, chlorosulfonyl isocyanate, trichloroacetyl isocyanate, trimethylsilyl isocyanate or the like, may be added to an alcohol compound solution having a (R)-configuration obtained by reducing a compound of Formula 1a in an organic solvent-for example, diethylether, tetrahydrofuran, 1,4-dioxane, acetonitrile, dichloromethane, chloroform or a mixture thereof, and reacted at a reaction temperature of about -50°C to 40°C, and then 1 to 20 equivalents of water may be sequentially added thereto without any purification to carry out hydrolysis.

In the method of using carbonyl compound-ammonia, 1 to 4 equivalents of carbonyl compound-for example, 1,1'-carbonyldiimidazole, carbamoyl chloride, N,N'-disuccinimidyl carbonate, phosgene, triphosgene, chloroformate or the like, are added to an alcohol compound solution in a (R)-configuration obtained by reducing a compound of Formula 1a in an organic solvent-for example, diethylether, tetrahydrofuran, 1,4-dioxane, acetonitrile, dichloromethane, chloroform or a mixture thereof, and then 1 to 10 equivalents of ammonia are sequentially added without purification at a reaction temperature of about -10°C to 70°C.

In addition, still another aspect of the present disclosure relates to a method for separating a compound of Formula 1a from a mixture comprising a compound of Formula 1a and a compound of 1b by heat treating the mixture comprising the compound of chemical formula 1a and the compound of Formula 1b to produce a compound of Formula 5 and removing this compound.

The heat treatment process is the same as described above.

In one embodiment, the removal of the compound of Formula 5 may be carried out by washing with an acidic aqueous solution. The acidic aqueous solution may be a solution of strong acid such as hydrochloric acid or sulfuric acid, or a solution of weak acid such as acetic acid or citric acid, but is not limited thereto.

Hereinafter, the present invention will be specifically described with reference to the following examples. However, these are provided only for better understanding of the present invention.

### Examples

### Example 1

Tetrazole (0.165 g) was dissolved in methanol (9 mL) and potassium carbonate (0.538 g) was added thereto at room temperature. The reaction product was stirred at room temperature for about 15 minutes. After confirming that carbon dioxide gas no longer occurs, n-butyl acetate (9 mL) was added, methanol was removed by distillation under reduced pressure, and n-butyl acetate was added. After adding 2-bromo-2'-chloroacetophenone (0.50 g) to the reaction solution, the reaction product was stirred at 50°C for 12 hours. After the temperature was adjusted to room temperature, it was confirmed that the selectivity of the compound of Formula 1b corresponding to Formula 1b was 40% and the selectivity of the compound of Formula 1a corresponding to Formula 1a was 60%, by HPLC. The HPLC conditions are as follows and are as used in the examples below:
The column was Phenomenex Luna C18, 5 µm, 4.6×250 mm and column temperature was 35°C. The mobile phase was acetonitrile : water at a ratio of 6 : 4 and contained 0.1% trifluoroacetic acid, and was flowed for 10 minutes at 2.0 mL/min under isocratic conditions. The wavelength was fixed at 245 nm, and the peak time position of the compound was 2.36 minutes for the compound of Formula 1a, 1.94 minutes for the compound of Formula 1b, and 4.01 minutes for 2-bromo-2'-chloroacetophenone.

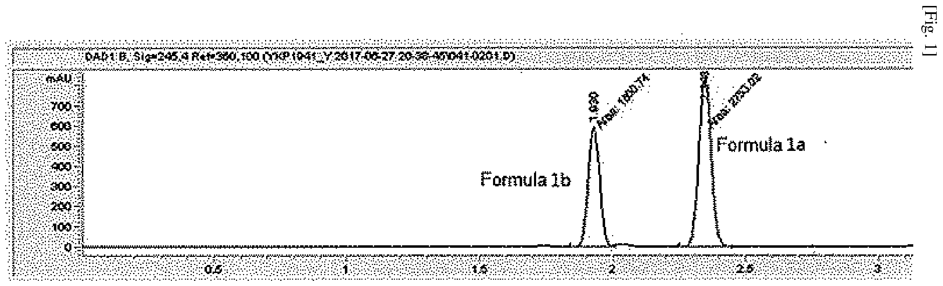

HPLC results are represented in Figure 1.

### Example 2

Tetrazole (0.165 g) was dissolved in n-butyl acetate (9 mL) and potassium carbonate (0.538 g) was added at room temperature. The reaction product was stirred at room temperature for about 24 hours. After confirming that carbon dioxide gas no longer occurred, 2-bromo-2'-chloroacetophenone (0.50 g) was added to the reaction solution and the reaction product was stirred at 50°C for 12 hours. After the temperature was adjusted to room temperature, it was confirmed that the selectivity of the compound of Formula 1a was 60% by HPLC.

### Example 3

Tetrazole (1.40 g) and potassium carbonate (1.38 g) were added to water (10 mL) and stirred at 100°C for about 1 hour under a reflux condition. The temperature was lowered to room temperature, water was distilled off, and the resultant was diluted in 20 mL of ethanol. The mixture was stirred at 80°C for 2 hours, and then the temperature was adjusted at room temperature. About 10 mL of ethanol was removed by distillation under reduced pressure and stirred for 2 hours, then filtered and dried under a nitrogen atmosphere to obtain the tetrazole potassium salt (1.70 g). The obtained tetrazole potassium salt (0.153 g) was added to n-butyl acetate (1.8 mL), 2-bromo-2'-chloroacetophenone (0.30 g) was added thereto, and the reaction mixture was stirred at 50°C for 12 hours. After the temperature was adjusted to room temperature, it was confirmed that the selectivity of the compound of Formula 1a was 62% by HPLC.

### Example 4

The tetrazole potassium salt (0.509 g) obtained in Example 3 was added to 2-methyl tetrahydrofuran (6 mL), 2-bromo-2'-chloroacetophenone (1.00 g) was added thereto, and the reaction mixture was stirred at 50°C for 22 hours. After the temperature was adjusted to room temperature, it was confirmed that the selectivity of the compound of Formula 1a was 57% by HPLC.

### Example 5

Tetrazole (1.40 g) and cesium carbonate (3.26 g) were added to water (10 mL) and stirred at 100°C for about 1 hour under a reflux condition. The temperature was lowered to room temperature, water was distilled off, and the reaction mixture was vacuum-dried to obtain tetrazole cesium salt (1.685 g). The obtained tetrazole cesium salt (0.285 g) was added to n-butyl acetate (1.8 mL), 2-bromo-2'-chloroacetophenone (0.30 g) was added thereto, and the reaction mixture was stirred at 50°C for 12 hours. After the temperature was adjusted to room temperature, it was confirmed that the selectivity of the compound of Formula 1a was 56% by HPLC.

### Example 6

Tetrazole (1.40 g) and sodium carbonate (0.68 g) were added to water (10 mL) and stirred at 100°C for about 1 hour under a reflux condition. The temperature was lowered to room temperature, water was distilled off, and the reaction mixture was vacuum-dried to obtain tetrazole sodium salt (1.53 g). The obtained tetrazole sodium salt (0.156 g) was added to n-butyl acetate (1.8 mL), 2-bromo-2'-chloroacetophenone (0.30 g) was added thereto, and the reaction mixture was stirred at 50°C for 12 hours. After the temperature was adjusted to room temperature, it was confirmed that the selectivity of the compound of Formula 1a was 63% by HPLC.

### Comparative Example 1

2-Bromo-2'-chloroacetophenone (86.0 g), potassium carbonate (30.5 g) and 35% tetrazole DMF solution (81.0 g) were added to ethyl acetate (245 mL) and stirred at 55°C for 2 hours. After the temperature was adjusted to room temperature, it was confirmed that the selectivity of the compound of Formula 1a was 42% by HPLC.

### Example 7

2-Bromo-2'-chloroacetophenone (13.0 g) was reacted with tetrazole potassium salt (6.62 g) and isopropyl acetate (117 mL), and then washed with diluted hydrochloric acid and brine to remove secondarily generated potassium bromide. The separated isopropyl acetate layer was completely concentrated, substituted with tert-butyl methyl ether, stirred under a reflux condition for about 1 hour, and then slowly cooled to 15°C. When the compound of Formula 1b was sufficiently precipitated, the resultant was filtered to obtain the compound of Formula 1b (4.1 g, including the compound of Formula 1a) as a solid. For reference, the filtrate was analyzed by HPLC, and it was confirmed that the filtrate is comprised of 6.0 g of the compound of Formula 1a and 0.74 g of the compound of Formula 1b.

Compounds of Formula 1b: ¹H NMR (CDCl3) 8.86 (s, 1H), 7.77 (d, 1H), 7. 40-7. 62 (m, 3H), 5.97 (s, 2H)

### Example 8

A solution of a mixture of 6.0 g of the compound of Formula 1a and 0.74 g of the compound of Formula 1b obtained as a filtrate in Example 7 was concentrated under reduced pressure to remove the solvent as much as possible, and while being substituted with isopropyl alcohol, the compound of Formula 1a was dissolved in isopropyl alcohol (45 mL), stirred for about 1 hour at 60°C, and slowly cooled to 10°C. The compound of Formula 1a was filtered after sufficient precipitation, washed twice with cooled isopropyl alcohol (13 mL) and once with n-heptane (26 mL) to obtain the compound of Formula 1a (5.55 g) as a solid with an HPLC purity of 94.2%.

Compounds of Formula 1a: ¹H NMR (CDCl3) 8.62 (s, 1H), 7.72 (d, 1H), 7. 35-7. 55 (m, 3H), 6.17 (s, 2H)

### Example 9

After reacting 2-bromo-2'-chloroacetophenone (17.1 g) with tetrazole potassium salt (8.71 g) and isopropyl acetate (130 mL), heptane (165 mL) was added to obtain the secondarily produced potassium bromide and the compound of Formula 1b as a solid at one time. After stirring at 60°C for 1 hour, the reaction mixture was slowly cooled to about 8.5°C. When the potassium bromide and the compound of Formula 1b were sufficiently precipitated, they were filtered to obtain potassium bromide and the compound of Formula 1b (total of 14 g) as solids. For reference, the filtrate was analyzed by HPLC, and it was confirmed that the filtrate is comprised of 9.5 g of the compound of Formula 1a and 1.4 g of the compound of Formula 1b.

### Example 10

A solution of a mixture of 9.5 g of the compound of Formula 1a and 1.4 g of the compound of Formula 1b-obtained as a filtrate in Example 9-was concentrated under reduced pressure to remove the solvent as much as possible and substituted with isopropyl alcohol. The compound of Formula 1a was stirred for about 1 hour at 60°C to dissolve in isopropyl alcohol (96 mL) and then slowly cooled to 10°C. When the compound of Formula 1a was sufficiently precipitated, the solid was filtered and washed twice with cold isopropyl alcohol (17 mL) and once with heptane (34 mL). The compound of Formula 1a (7.6 g) was obtained as a solid.

### Example 11

A mixture in which 2'-chlorophenyl 2-tetrazol-2-yl ketone (0.3 g) and 2'-chlorophenyl 2-tetrazole-1-yl ketone (0.2 g) were dissolved in isopropyl acetate (3 mL) was heated at 150°C for 24 hours, the temperature was adjusted to room temperature, and a pyrolysis ratio was confirmed through HPLC. It was confirmed that 99.9% of 2'-chlorophenyl 2-tetrazol-1-yl was decomposed and transformed to 5-(2-chlorophenyl)oxazol-2-amine, and 88.2% of 2'-chlorophenyl 2-tetrazol-2-yl remained without decomposition by HPLC. The produced 5-(2-chlorophenyl)oxazol-2-amine was washed with 1N HCl to remove this compound.

The column was Phenomenex Luna C18, 5 µm, 4.6×250 mm and column temperature was 35°C. The mobile phase was acetonitrile : water at a ratio of 6 : 4 and contained 0.1% trifluoroacetic acid, and was flowed for 10 minutes at 2.0 mL/min under isocratic conditions. The wavelength was fixed at 245 nm, and the peak time position of the compound was 2.36 minutes for the compound of Formula 1a, 1.94 minutes for the compound of Formula 1b, and 1.23 minutes for 5-(2-chlorophenyl)oxazol-2-amine of Formula 5.
5-(2-Chlorophenyl)oxazol-2-amine: LC-MS [M+H]=195.0 g/mol

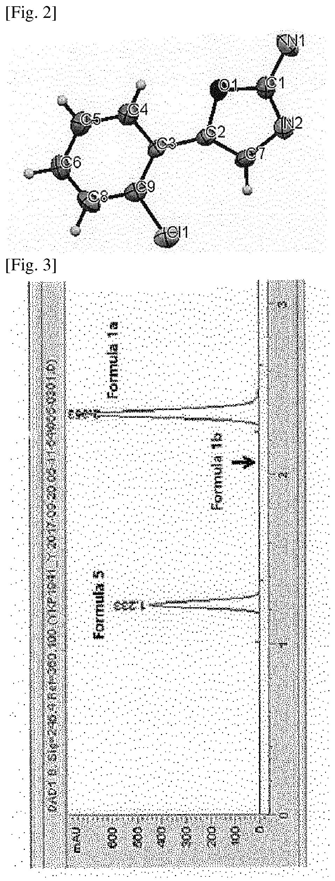

The structure of 5-(2-chlorophenyl)oxazol-2-amine was confirmed by ORTEP image and represented in Figure 2.

HPLC results are represented in Figure 3.

### Example 12

A mixture in which 2'-chlorophenyl 2-tetrazol-2-yl ketone (0.3 g) and 2'-chlorophenyl 2-tetrazole-1-yl ketone (0.2 g) were dissolved in isopropanol (3 mL) was flowed in a tube-type continuous reactor set at 210°C for 20 minutes, and then the temperature was adjusted to room temperature, and a pyrolysis ratio was confirmed through HPLC. It was confirmed that 99.9% of 2'-chlorophenyl 2-tetrazol-1-yl was decomposed and transformed to 5-(2-chlorophenyl)oxazol-2-amine, and 90% of 2'-chlorophenyl 2-tetrazol-2-yl remained without decomposition.

## Claims

1. A method for preparing a compound of Formula 1a, comprising: 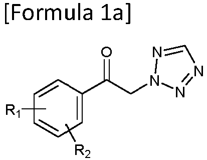
reacting a compound of Formula 3 with a base in a reaction solvent to obtain a salt of a compound of Formula 3; and
reacting a compound of Formula 2 with the salt of the compound of Formula 3: 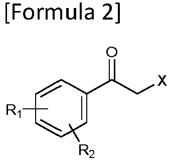 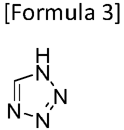
wherein
R₁ and R₂ are each independently selected from the group consisting of hydrogen, halogen, perfluoroalkyl having 1 to 8 carbon atoms, alkyl having 1 to 8 carbon atoms, thioalkoxy having 1 to 8 carbon atoms, and alkoxy having 1 to 8 carbon atoms; and X is a leaving group selected from halides and sulfonates.

2. The method according to claim 1, which further comprises:
purifying the reaction product of the salt of the compound of Formula 3 and the compound of Formula 2, wherein the purifying step comprises a crystallizing process or a heat treatment process;
wherein the heat treatment process is carried out at a reaction temperature of 100°C to 250°C and a reaction time of from 10 minutes to 40 hours.

3. The method according to claim 1 or 2, wherein the salt is one or more selected from the group consisting of a lithium salt, a sodium salt, a potassium salt and a cesium salt.

4. The method according to claim 1, wherein the base is an inorganic base or an organic base; preferably
wherein the inorganic base is a metal hydroxide or a metal carbonate, and the organic base is an amine compound; and preferably
wherein the metal hydroxide is selected from lithium hydroxide, sodium hydroxide and potassium hydroxide; the metal carbonate is selected from lithium carbonate, sodium carbonate, potassium carbonate and cesium carbonate; and the amine compound is selected from triethylamine and diisopropylethylamine.

5. The method according to claim 1, wherein the compound of Formula 3 is reacted with the base in the reaction solvent, and then the salt of the compound of Formula 3 is separated from the reaction mixture of the compound of Formula 3 and the base; preferably
wherein the separated salt of the compound of Formula 3 is reacted with the compound of Formula 2.

6. The method according to claim 1, wherein after reacting the compound of Formula 3 with the base, the compound of Formula 2 is added to the reaction product to react with the salt of the compound of Formula 3.

7. The method according to claim 2, wherein the crystallizing process comprises a first crystallizing process and a second crystallizing process; preferably
wherein a solvent of the first crystallizing process is selected from the group consisting of water, C₁-C₄ lower alcohol, diethyl ether, tert-butyl methyl ether, isopropyl ether, pentane, hexane, cyclohexane, heptane and a mixture thereof; or preferably
wherein a solvent of the second crystallizing process is selected from the group consisting of acetone, acetonitrile, tetrahydrofuran, 2-methyl tetrahydrofuran, ethyl acetate, isopropyl acetate, n-butyl acetate, dichloromethane, chloroform, 1,4-dioxane, C₁-C₄ lower alcohol and a mixture thereof.

8. The method according to claim 1, wherein the leaving group is selected from the group consisting of chloride, bromide, mesylate, tosylate and 4-nitrophenyl sulfonate.

9. The method according to claim 2, wherein the purifying step comprises a heat treatment process of the mixture comprising the compound of Formula 1a and the compound of Formula 1b: 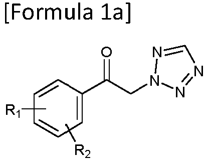 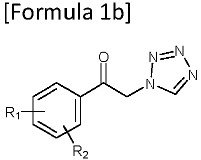 wherein
R₁ and R₂ are each independently selected from the group consisting of hydrogen, halogen, perfluoroalkyl having 1 to 8 carbon atoms, alkyl having 1 to 8 carbon atoms, thioalkoxy having 1 to 8 carbon atoms, and alkoxy having 1 to 8 carbon atoms.

10. The method according to claim 9, wherein the heat treatment process is a step of transforming the compound of Formula 1b to a compound of Formula 5: 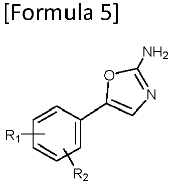 wherein R₁ and R₂ are the same as defined in claim 9.

11. The method according to claim 2 or 9, wherein the heat treatment process is carried out in the presence of a solvent; preferably
wherein the solvent is acetone, acetonitrile, tetrahydrofuran, 2-methyl tetrahydrofuran, ethyl acetate, isopropyl acetate, n-butyl acetate, dichloromethane, chloroform, 1,4-dioxane, C₁-C₄ lower alcohol or a mixture thereof.

12. The method according to claim 2 or 9, wherein the heat treatment process is carried out at a pressure of 1 atmosphere to 50 atmosphere.

13. The method according to claim 2 or 9, which further comprises a step of washing a product of the heat treatment process with an acidic aqueous solution; preferably wherein the acidic aqueous solution is an aqueous solution of hydrochloric acid, sulfuric acid, acetic acid or citric acid.

14. The method according to claim 13, wherein the compound of Formula 5 is removed by the step of washing with the acidic aqueous solution: 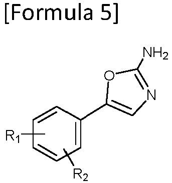 wherein R₁ and R₂ are the same as defined in claim 9.

## Patentansprüche

1. Ein Verfahren zur Herstellung einer Verbindung der Formel 1a, umfassend: 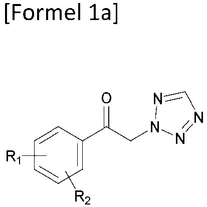
Umsetzen einer Verbindung der Formel 3 mit einer Base in einem Reaktionslösungsmittel, um ein Salz einer Verbindung der Formel 3 zu erhalten; und Umsetzen einer Verbindung der Formel 2 mit dem Salz der Verbindung der Formel 3: 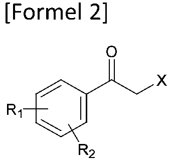 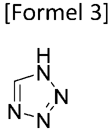
wobei
R₁ und R₂ jeweils unabhängig ausgewählt sind aus der Gruppe bestehend aus Wasserstoff, Halogen, Perfluoralkyl mit 1 bis 8 Kohlenstoffatomen, Alkyl mit 1 bis 8 Kohlenstoffatomen, Thioalkoxy mit 1 bis 8 Kohlenstoffatomen und Alkoxy mit 1 bis 8 Kohlenstoffatomen; und
X eine Abgangsgruppe, ausgewählt aus Halogeniden und Sulfonaten, ist.

2. Das Verfahren gemäß Anspruch 1, welches ferner umfasst:
Reinigen des Reaktionsproduktes des Salzes der Verbindung der Formel 3 und der Verbindung der Formel 2, wobei der Reinigungsschritt ein Kristallisationsverfahren oder ein Wärmebehandlungsverfahren umfasst;
wobei das Wärmebehandlungsverfahren bei einer Reaktionstemperatur von 100°C bis 250°C und einer Reaktionszeit von 10 Minuten bis 40 Stunden durchgeführt wird.

3. Das Verfahren gemäß Anspruch 1 oder 2, wobei das Salz eines oder mehrere, ausgewählt aus der Gruppe bestehend aus einem Lithiumsalz, einem Natriumsalz, einem Kaliumsalz und einem Cäsiumsalz, ist.

4. Das Verfahren gemäß Anspruch 1, wobei die Base eine anorganische Base oder eine organische Base ist; vorzugsweise
wobei die anorganische Base ein Metallhydroxid oder ein Metallcarbonat ist und die organische Base eine Aminverbindung ist; und vorzugsweise
wobei das Metallhydroxid ausgewählt ist aus Lithiumhydroxid, Natriumhydroxid und Kaliumhydroxid; das Metallcarbonat ausgewählt ist aus Lithiumcarbonat, Natriumcarbonat, Kaliumcarbonat und Cäsiumcarbonat; und die Aminverbindung ausgewählt ist aus Triethylamin und Diisopropylethylamin.

5. Das Verfahren gemäß Anspruch 1, wobei die Verbindung der Formel 3 mit der Base in dem Reaktionslösungsmittel umgesetzt wird und dann das Salz der Verbindung der Formel 3 von dem Reaktionsgemisch der Verbindung der Formel 3 und der Base abgetrennt wird; vorzugsweise
wobei das abgetrennte Salz der Verbindung der Formel 3 mit der Verbindung der Formel 2 umgesetzt wird.

6. Das Verfahren gemäß Anspruch 1, wobei nach dem Umsetzen der Verbindung der Formel 3 mit der Base die Verbindung der Formel 2 zu dem Reaktionsprodukt zugegeben wird, um mit dem Salz der Verbindung der Formel 3 zu reagieren.

7. Das Verfahren gemäß Anspruch 2, wobei das Kristallisationsverfahren ein erstes Kristallisationsverfahren und ein zweites Kristallisationsverfahren umfasst; vorzugsweise
wobei ein Lösungsmittel des ersten Kristallisationsverfahrens ausgewählt ist aus der Gruppe bestehend aus Wasser, C₁-C₄-Niederalkohol, Diethylether, tert-Butylmethylether, Isopropylether, Pentan, Hexan, Cyclohexan, Heptan und einem Gemisch davon; oder vorzugsweise
wobei ein Lösungsmittel des zweiten Kristallisationsverfahrens ausgewählt ist aus der Gruppe bestehend aus Aceton, Acetonitril, Tetrahydrofuran, 2-Methyltetrahydrofuran, Ethylacetat, Isopropylacetat, n-Butylacetat, Dichlormethan, Chloroform, 1,4-Dioxan, C₁-C₄-Niederalkohol und einem Gemisch davon.

8. Das Verfahren gemäß Anspruch 1, wobei die Abgangsgruppe ausgewählt ist aus der Gruppe bestehend aus einem Chlorid, Bromid, Mesylat, Tosylat und 4-Nitrophenylsulfonat.

9. Das Verfahren gemäß Anspruch 2, wobei der Reinigungsschritt ein Wärmebehandlungsverfahren des Gemisches umfassend die Verbindung der Formel 1a und die Verbindung der Formel 1b umfasst: 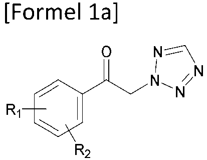 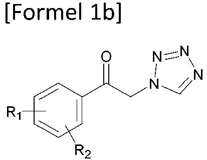 wobei
R₁ und R₂ jeweils unabhängig ausgewählt sind aus der Gruppe bestehend aus Wasserstoff, Halogen, Perfluoralkyl mit 1 bis 8 Kohlenstoffatomen, Alkyl mit 1 bis 8 Kohlenstoffatomen, Thioalkoxy mit 1 bis 8 Kohlenstoffatomen und Alkoxy mit 1 bis 8 Kohlenstoffatomen.

10. Das Verfahren gemäß Anspruch 9, wobei das Wärmebehandlungsverfahren ein Schritt des Umwandelns der Verbindung der Formel 1b in eine Verbindung der Formel 5 ist: 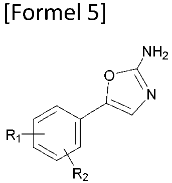 wobei R₁ und R₂ wie vorstehend in Anspruch 9 definiert sind.

11. Das Verfahren gemäß Anspruch 2 oder 9, wobei das Wärmebehandlungsverfahren in Gegenwart eines Lösungsmittels durchgeführt wird; vorzugsweise wobei das Lösungsmittel Aceton, Acetonitril, Tetrahydrofuran, 2-Methyltetrahydrofuran, Ethylacetat, Isopropylacetat, n-Butylacetat, Dichlormethan, Chloroform, 1,4-Dioxan, C₁-C₄-Niederalkohol oder ein Gemisch davon ist.

12. Das Verfahren gemäß Anspruch 2 oder 9, wobei das Wärmebehandlungsverfahren bei einem Druck von 1 Atmosphäre bis 50 Atmosphären durchgeführt wird.

13. Das Verfahren gemäß Anspruch 2 oder 9, welches ferner einen Schritt des Waschens eines Produkts des Wärmebehandlungsverfahrens mit einer sauren wässrigen Lösung umfasst; vorzugsweise
wobei die saure wässrige Lösung eine wässrige Lösung aus Hypochlorsäure, Schwefelsäure, Essigsäure oder Zitronensäure ist.

14. Das Verfahren gemäß Anspruch 13, wobei die Verbindung der Formel 5 durch den Schritt des Waschens mit der sauren wässrigen Lösung entfernt wird: 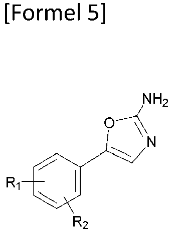 wobei R₁ und R₂ wie vorstehend in Anspruch 9 definiert sind.

## Revendications

1. Méthode de préparation d'un composé de Formule 1a, comprenant : 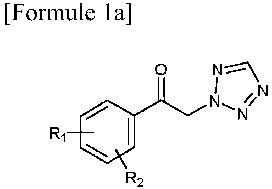
la réaction d'un composé de Formule 3 avec une base dans un solvant de réaction afin d'obtenir un sel du composé de Formule 3 ; et
la réaction d'un composé de Formule 2 avec le sel du composé de Formule 3 ; 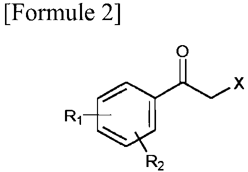 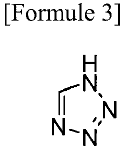
dans lesquelles
R₁ et R₂ sont chacun indépendamment choisis dans le groupe constitué par un hydrogène, un halogène, un perfluoroalkyle ayant 1 à 8 atomes de carbone, un alkyle ayant 1 à 8 atomes de carbone, un thioalcoxy ayant 1 à 8 atomes de carbone et un alcoxy ayant 1 à 8 atomes de carbone ; et
X est un groupe partant choisi parmi les halogénures et les sulfonates.

2. Méthode selon la revendication 1, qui comprend en outre :
la purification du produit de réaction du sel du composé de Formule 3 et du composé de Formule 2, dans laquelle l'étape de purification comprend un procédé de cristallisation ou un procédé de traitement thermique ;
dans laquelle le procédé de traitement thermique est réalisé à une température de réaction de 100°C à 250°C et à un temps de réaction de 10 minutes à 40 heures.

3. Méthode selon la revendication 1 ou 2, dans laquelle le sel est un ou plusieurs éléments choisis dans le groupe constitué par un sel de lithium, un sel de sodium, un sel de potassium et un sel de césium.

4. Méthode selon la revendication 1, dans laquelle la base est une base inorganique ou une base organique ; de préférence
dans laquelle la base inorganique est un hydroxyde de métal ou un carbonate de métal, et la base organique est un composé amine ; et de préférence
dans laquelle l'hydroxyde de métal est choisi parmi l'hydroxyde de lithium, l'hydroxyde de sodium et l'hydroxyde de potassium ; le carbonate de métal est choisi parmi le carbonate de lithium, le carbonate de sodium, le carbonate de potassium et le carbonate de césium ; et le composé amine est choisi parmi la triéthylamine et la diisopropyléthylamine.

5. Méthode selon la revendication 1, dans laquelle le composé de Formule 3 est mis à réagir avec la base dans le solvant de réaction, et puis le sel du composé de Formule 3 est séparé du mélange réactionnel du composé de Formule 3 et de la base ; de préférence
dans laquelle le sel séparé du composé de Formule 3 est mis à réagir avec le composé de Formule 2.

6. Méthode selon la revendication 1, dans laquelle après réaction du composé de Formule 3 avec la base, le composé de Formule 2 est ajouté au produit de réaction pour réagir avec le sel du composé de Formule 3.

7. Méthode selon la revendication 2, dans laquelle le procédé de cristallisation comprend un premier procédé de cristallisation et un deuxième procédé de cristallisation ; de préférence
dans laquelle un solvant du premier procédé de cristallisation est choisi dans le groupe constitué par l'eau, un alcool inférieur en C₁ à C₄, l'éther diéthylique, le méthyl tert-butyl éther, l'éther isopropylique, le pentane, l'hexane, le cyclohexane, l'heptane et un mélange de ceux-ci ; ou de préférence
dans laquelle un solvant du deuxième procédé de cristallisation est choisi dans le groupe constitué par l'acétone, l'acétonitrile, le tétrahydrofurane, le 2-méthyl-tétrahydrofurane, l'acétate d'éthyle, l'acétate d'isopropyle, l'acétate de n-butyle, le dichlorométhane, le chloroforme, le 1,4-dioxane, un alcool inférieur en C₁ à C₄, et un mélange de ceux-ci.

8. Méthode selon la revendication 1, dans laquelle le groupe partant est choisi dans le groupe constitué par un chlorure, un bromure, un mésylate, un tosylate et le 4-nitrophényl sulfonate.

9. Méthode selon la revendication 2, dans laquelle l'étape de purification comprend un procédé de traitement thermique du mélange comprenant le composé de Formule 1a et le composé de Formule 1b : 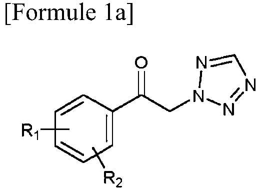 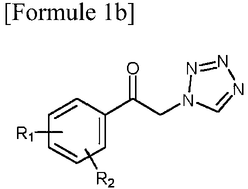 dans lesquelles
R₁ et R₂ sont chacun indépendamment choisis dans le groupe constitué par un hydrogène, un halogène, un perfluoroalkyle ayant 1 à 8 atomes de carbone, un alkyle ayant 1 à 8 atomes de carbone, un thioalcoxy ayant 1 à 8 atomes de carbone et un alcoxy ayant 1 à 8 atomes de carbone.

10. Méthode selon la revendication 9, dans laquelle le procédé de traitement thermique est une étape de transformation du composé de Formule 1b en un composé de Formule 5 : 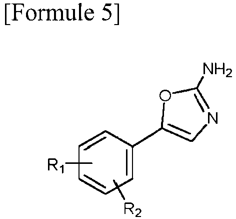 dans laquelle R₁ et R₂ sont tels que définis selon la revendication 9.

11. Méthode selon la revendication 2 ou 9, dans laquelle le procédé de traitement thermique est réalisé en présence d'un solvant ; de préférence
dans laquelle le solvant est l'acétone, l'acétonitrile, le tétrahydrofurane, le 2-méthyl-tétrahydrofurane, l'acétate d'éthyle, l'acétate d'isopropyle, l'acétate de n-butyle, le dichlorométhane, le chloroforme, le 1,4-dioxane, un alcool inférieur en C₁ à C₄, ou un mélange de ceux-ci.

12. Méthode selon la revendication 2 ou 9, dans laquelle le procédé de traitement thermique est réalisé à une pression de 1 atmosphère à 50 atmosphères.

13. Méthode selon la revendication 2 ou 9, qui comprend en outre une étape de lavage d'un produit du procédé de traitement thermique avec une solution aqueuse acide ; de préférence
dans laquelle la solution aqueuse acide est une solution aqueuse d'acide chlorhydrique, d'acide sulfurique, d'acide acétique ou d'acide citrique.

14. Méthode selon la revendication 13, dans laquelle le composé de Formule 5 est éliminé par l'étape de lavage avec la solution aqueuse acide : 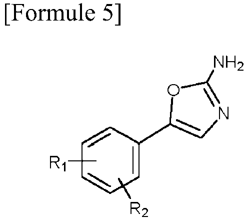 dans laquelle R₁ et R₂ sont tels que définis selon la revendication 9.
